(19) Europäisches Patentamt
European Patent Office
Office européen des brevets

(11) EP 1 187 627 B1

(12) **EUROPEAN PATENT SPECIFICATION**

(45) Date of publication and mention of the grant of the patent:
**17.09.2003 Bulletin 2003/38**

(21) Application number: **00935374.9**

(22) Date of filing: **01.06.2000**

(51) Int Cl.[7]: **A61K 38/17**, A61P 13/00, A61P 15/10, A61P 17/14, A61P 25/08, A61P 25/14, A61P 25/22, A61P 25/28, A61P 25/30

(86) International application number:
**PCT/GB00/02107**

(87) International publication number:
**WO 00/072863 (07.12.2000 Gazette 2000/49)**

(54) **USES OF H-TREK-1 POLYPEPTIDES AND POLYNUCLEOTIDES ENCODING THEM**

VERWENDUNGEN VON H-TREK-1 POLYPEPTIDE, SOWIE DER DAFÜR KODIERENDEN POLYNUKLEOTIDEN

UTILISATIONS DE POLYPEPTIDES ET POLYNUCLEOTIDES H-TREK-1

(84) Designated Contracting States:
**AT BE CH CY DE DK ES FI FR GB GR IE IT LI LU MC NL PT SE**

(30) Priority: **01.06.1999 GB 9912733**

(43) Date of publication of application:
**20.03.2002 Bulletin 2002/12**

(73) Proprietor: **SmithKline Beecham plc**
**Brentford, Middlesex TW8 9GS (GB)**

(72) Inventors:
- **HERVIEU, Guillaume Jean, SmithKline Beecham Pharm. Harlow, Essex CM19 5AW (GB)**
- **MEADOWS, Helen Jane, SmithKline Beecham Pharma. Harlow, Essex CM19 5AW (GB)**
- **RANDALL, Andrew David, SmithKline Beecham Pharma. Harlow, Essex CM19 5AW (GB)**

(74) Representative: **Crump, Julian Richard John et al**
**FJ Cleveland,**
**40-43 Chancery Lane**
**London WC2A 1JQ (GB)**

(56) References cited:
**WO-A-99/37762**

- **M. FINK ET AL.: "Cloning, functional expression and brain localization of a novel unconventional outward rectifier K+ channel" THE EMBO JOURNAL, vol. 15, no. 24, 1996, pages 6854-6862, XP002085602**

EP 1 187 627 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

SEQUENCE LISTING

<110> SmithKline Beecham plc
<120> New Use
<130> P32320
<160> 2
<170> FastSEQ for Windows Version 3.0
<210> 1
<211> 1246
<212> DNA
<213> Homo sapiens
<400> 1

```
gaataagaat ggcggcacct gacttgctgg atcctaaatc tgccgctcag aactccaaac      60
cgaggctctc gttttccacg aaacccacag tgcttgcttc ccgggtggag agtgacacga     120
ccattaatgt tatgaaatgg aagacggtct ccacgatatt cctggtggtt gtcctctatc     180
tgatcatcgg agccaccgtg ttcaaagcat tggagcagcc tcatgagatt tcacagagga     240
ccaccattgt gatccagaag caaacattca tatcccaaca ttcctgtgtc aattcgacgg     300
agctggatga actcattcag caaatagtgg cagcaataaa tgcagggatt ataccgttag     360
gaaacacctc caatcaaatc agtcactggg atttgggaag ttccttcttc tttgctggca     420
ctgttattac aaccatagga tttggaaaca tctcaccacg cacagaaggc ggcaaaatat     480
tctgtatcat ctatgcctta ctgggaattc cctctttgg ttttctcttg gctggagttg     540
gagatcagct aggcaccata tttggaaaag gaattgccaa agtggaagat acgtttatta     600
agtggaatgt tagtcagacc aagattcgca tcatctcaac aatcatattt atactatttg     660
gctgtgtact ctttgtggct ctgcctgcga tcatattcaa acacatagaa ggctggagtg     720
ccctggacgc catttatttt gtggttatca ctctaacaac tattggattt ggtgactacg     780
ttgcaggtgg atccgatatt gaatatctgg acttctataa gcctgtcgtg tggttctgga     840
tccttgtagg gcttgcttac tttgctgctg tcctgagcat gattggagat tggctccgag     900
tgatatctaa aaagacaaaa gaagaggtgg gagagttcag agcacacgct gctgagtgga     960
cagccaacgt cacagccgaa ttcaaagaaa ccaggaggcg actgagtgtg gagatttatg    1020
acaagttcca gcgggccacc tccatcaagc ggaagctctc ggcagaactg gctggaaacc    1080
acaatcagga gctgactcct tgtaggagga ccctgtcagt gaaccacctg accagcgaga    1140
gggatgtctt gcctccctta ctgaagactg agagtatcta tctgaatggt ttgacgccac    1200
actgtgctgg tgaagagatt gctgtgattg agaacatcaa atagcc                   1246
```

<210> 2
<211> 411
<212> PRT

<213> Homo sapiens
<400> 2

```
Met Ala Ala Pro Asp Leu Leu Asp Pro Lys Ser Ala Ala Gln Asn Ser
 1               5                  10                  15
Lys Pro Arg Leu Ser Phe Ser Thr Lys Pro Thr Val Leu Ala Ser Arg
            20                  25                  30
Val Glu Ser Asp Thr Thr Ile Asn Val Met Lys Trp Lys Thr Val Ser
        35                  40                  45
Thr Ile Phe Leu Val Val Val Leu Tyr Leu Ile Ile Gly Ala Thr Val
    50                  55                  60
Phe Lys Ala Leu Glu Gln Pro His Glu Ile Ser Gln Arg Thr Thr Ile
65                  70                  75                  80
Val Ile Gln Lys Gln Thr Phe Ile Ser Gln His Ser Cys Val Asn Ser
                85                  90                  95
Thr Glu Leu Asp Glu Leu Ile Gln Gln Ile Val Ala Ala Ile Asn Ala
            100                 105                 110
Gly Ile Ile Pro Leu Gly Asn Thr Ser Asn Gln Ile Ser His Trp Asp
        115                 120                 125
Leu Gly Ser Ser Phe Phe Phe Ala Gly Thr Val Ile Thr Thr Ile Gly
    130                 135                 140
Phe Gly Asn Ile Ser Pro Arg Thr Glu Gly Gly Lys Ile Phe Cys Ile
145                 150                 155                 160
Ile Tyr Ala Leu Leu Gly Ile Pro Leu Phe Gly Phe Leu Leu Ala Gly
                165                 170                 175
Val Gly Asp Gln Leu Gly Thr Ile Phe Gly Lys Gly Ile Ala Lys Val
            180                 185                 190
Glu Asp Thr Phe Ile Lys Trp Asn Val Ser Gln Thr Lys Ile Arg Ile
        195                 200                 205
Ile Ser Thr Ile Ile Phe Ile Leu Phe Gly Cys Val Leu Phe Val Ala
    210                 215                 220
Leu Pro Ala Ile Ile Phe Lys His Ile Glu Gly Trp Ser Ala Leu Asp
225                 230                 235                 240
Ala Ile Tyr Phe Val Val Ile Thr Leu Thr Thr Ile Gly Phe Gly Asp
                245                 250                 255
Tyr Val Ala Gly Gly Ser Asp Ile Glu Tyr Leu Asp Phe Tyr Lys Pro
            260                 265                 270
Val Val Trp Phe Trp Ile Leu Val Gly Leu Ala Tyr Phe Ala Ala Val
        275                 280                 285
Leu Ser Met Ile Gly Asp Trp Leu Arg Val Ile Ser Lys Lys Thr Lys
    290                 295                 300
Glu Glu Val Gly Glu Phe Arg Ala His Ala Ala Glu Trp Thr Ala Asn
305                 310                 315                 320
Val Thr Ala Glu Phe Lys Glu Thr Arg Arg Arg Leu Ser Val Glu Ile
                325                 330                 335
Tyr Asp Lys Phe Gln Arg Ala Thr Ser Ile Lys Arg Lys Leu Ser Ala
            340                 345                 350
```

```
Glu Leu Ala Gly Asn His Asn Gln Glu Leu Thr Pro Cys Arg Arg Thr
        355                 360                 365
Leu Ser Val Asn His Leu Thr Ser Glu Arg Asp Val Leu Pro Pro Leu
    370                 375                 380
Leu Lys Thr Glu Ser Ile Tyr Leu Asn Gly Leu Thr Pro His Cys Ala
385                 390                 395                 400
Gly Glu Glu Ile Ala Val Ile Glu Asn Ile Lys
                405                 410
```

## Description

### Field of the Invention

This invention relates to new uses for polynucleotides and polypeptides encoded by them, to their use in therapy and in identifying compounds which may be agonists or antagonists which are potentially useful in therapy.

### Summary of the Invention

In one aspect the invention relates to new uses of h-TREK1 polynucleotides and polypeptides disclosed in WO99/37762 (SmithKline Beecham). Such uses include the treatment of epilepsy, sleep-related disorders, the induction of sleep, cognitive dysfunction, attention deficit disorder, addiction, anxiety / phobia, dyskinesias including Parkinson's and Huntington's chorea, cerebral palsy, incontinence, erectile dysfunction, alopecia, hereinafter referred to as "the Diseases".

In a further aspect, the invention describes the identification of agonists and antagonists using the materials provided by the invention and the use of these agonists and antagonists the manufacture of a medicament for treating conditions associated with h-TREK1 imbalance or mutation.

### Description of the Invention

The h-TREK1 polypeptides of the present invention, disclosed in WO99/37762 (SmithKline Beecham), are members of the 2P domain potassium channel family of polypeptides which play a part in the control of resting membrane potential. Modulation of these channels will therefore affect neuronal excitability, thereby leading to a modulation of neurotransmitter release and the activity of neuronal networks. Such modulation therefore may be useful for the treatment of certain neurological conditions.

The present invention is based on the finding that h-TREK1 channels are present on both projection neurones and interneurones within the central nervous system and are highly expressed on a number of GABA-ergic neurones. For example h-TREK1 is highly expressed in the GABA-ergic cells of the reticular thalamic nucleus. The reticular thalamic nucleus plays an important role in the transition from wakefulness to sleep by inhibiting via GABA-ergic projections the activity of other thalamic nuclei. Thus an h-TREK1 channel modulator, preferably an antagonist, could alter alertness and may facilitate the transition from wakefulness to sleep or *vice-versa*. Modulators of this polypeptide are therefore of interest because of their potential for use in both the induction of sleep and the treatment of other sleep-related disorders.

h-TREK1 is also shown to be present in cortical and hippocampal neurones, in particular GABA-ergic interneurones. Neuronal cells and circuits in these two areas have well characterized prominent roles in cognition, learning and memory. Furthermore, in pathological conditions that produce cognitive deficits, substantial changes are often observed in the anatomy and/or gene expression patterns in cortex and hippocampus. At the cellular level the changes that lead to learning and memory are believed to primarily arise from alterations to the electrical excitability of hippocampal and cortical neuronal circuits. Given the role of 2-P-domain potassium channels such as h-TREK1 in the determination of resting membrane potential, and consequently neuronal excitability, a compound that modulates h-TREK1 activity, preferably an agonist, may therefore be a useful agent for producing alterations, for example enhancement, in cognitive function and/or for the treatment of cognitive dysfunction associated with various disease states such as Alzheimer's disease.

h-TREK1 protein is also present within nuclei of the basal ganglia. These include the sub-thalamic nucleus, substantia nigra pars reticulata and caudate putamen. These structures play a prominent role in a range of neurological disorders characterized by a range of dyskinesias such as tremor (e.g. that associated with Parkinson's Disease), chorea (e.g that associated with Huntington's disease) athetosis (e.g. that associated with cerebral palsy) and ballsim. These dyskinesias are associated with changes to electrical activity and synaptic signaling in these brain areas and have close associations with the neurotransmitter dopamine and GABA. Given the localization of h-TREK1 in nuclei of the basal ganglia, its prominent role in determining electrical excitability and the frequent co-localization of h-TREK1 on GABA-ergic interneurones a modulator of h-TREK1 has potential to act as a therapeutic agent in the treatment of a range of dyskinesias, including those associated with Parkinson's and Huntington's diseases.

It is well known in the art that highly efficaceous anxiolytic compounds are those which facilitate GABA-ergic function. The present finding that h-TREK1 is highly expressed on GABA-ergic interneurones together with localisation of h-TREK1 to the amygdala, a classical seat of anxiety, suggests that h-TREK1 modulators have potential use in the treatment of anxiety.

The term "modulators" as used herein is understood to encompass all molecules and compounds which affect the activity of an h-TREK1 channel, and includes all agonists, antagonists and inhibitors. It will be understood by the skilled person that both agonists and antagonists will be useful in the treatment of sleep-related disorders depending on whether the required effect is to facilitate the induction of sleep, where an antagonist would be more appropriate, or to prevent inappropriate and/or excessive sleep (for example narcolepsy) where an agonist would be more appropriate. Similarly an h-TREK1 antagonist which would increase excitability of GABA-ergic cells would be expected to be more useful (than an agonist) for the treatment of epilepsy or anxiety, whereas an h-TREK1 agonist may be more appropriate for use in the treatment of cognitive dysfunction.

Therefore in a first aspect, the present invention relates to the use of a compound selected from:

(a) an h-TREK1 polypeptide;
(b) a compound which activates an h-TREK1 polypeptide; or
(c) a compound which inhibits an h-TREK1 polypeptide; or .
(d) a polynucleotide encoding an h-TREK1 polypeptide,

for the manufacture of a medicament for treating:

(i) epilepsy;
(ii) sleep-related disorders;
(iii) cognitive dysfunction;
(iv) attention deficit disorder;
(v) addiction;
(vi) anxiety / phobia;
(vii) dyskinesias including Parkinson's and Huntington's chorea;
(viii) cerebral palsy;
(ix) incontinence;
(x) erectile dysfunction; or
(xi) alopecia.

In a preferred aspect the sleep-related disorder is one of excessive and/or inappropriate wakefulness, for example insomnia, wherein the treatment comprises the use of a compound selected from (a) to (d) above, preferably (c), to facilitate the induction of sleep.

In a further preferred embodiment the invention relates to the use of a compound selected from (a) to (d) above, preferably (c) for the treatment of epilepsy.

In a still further preferred embodiment the invention relates to the use of a compound selected from (a) to (d) above, preferably (b) for the treatment of cognitive dysfunction wherein said treatment results in cognitive enhancement.

Such h-TREK1 polypeptides include isolated polypeptides comprising an amino acid sequence which has at least 95% identity, preferably at least 97-99% identity, to that of SEQ ID NO:2 over the entire length of SEQ ID NO:2. Such polypeptides include those comprising the amino acid of SEQ ID NO:2.

Further polypeptides of the present invention include isolated polypeptides in which the amino acid sequence has at least 95% identity, preferably at least 97-99% identity, to the amino acid sequence of SEQ ID NO:2 over the entire length of SEQ ID NO:2. Such polypeptides include the polypeptide of SEQ ID NO:2.

Further peptides of the present invention include isolated polypeptides encoded by a polynucleotide comprising the sequence contained in SEQ ID NO:1.

The polypeptides of the present invention may be in the form of the "mature" protein or may be a part of a larger protein such as a precursor or a fusion protein. It is often advantageous to include an additional amino acid sequence which contains secretory or leader sequences, pro-sequences, sequences which aid in purification such as multiple histidine residues, or an additional sequence for stability during recombinant production.

The present invention also includes variants of the aforementioned polypeptides, that is polypeptides that vary from the referents by conservative amino acid substitutions, whereby a residue is substituted by another with like characteristics. Typical such substitutions are among Ala, Val, Leu and Ile; among Ser and Thr; among the acidic residues Asp and Glu; among Asn and Gln; and among the basic residues Lys and Arg; or aromatic residues Phe and Tyr. Particularly preferred are variants in which several, 5-10, 1-5, 1-3, 1-2 or 1 amino acids are substituted, deleted, or added in any combination.

Polypeptides of the present invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

The h-TREK1 polypeptides of the invention have at least one biological acitivity, for example that of controlling the resting membrane potential of neurones. These properties are hereinafter referred to as "h-TREK1 activity" or h-TREK1 polypeptide activity" or "biological activity of h-TREK1". Also included amongst these activities are antigenic and immunogenic activities of said h-TREK1 polypeptides, in particular the antigenic and immunogenic activities of the polypeptide of SEQ ID NO:2. Preferably, a polypeptide of the present invention exhibits at least one biological acitivity of h-TREK1.

In a further aspect, the present invention relates to h-TREK1 polynucleotides. Such polynucleotides include isolated polynucleotides comprising a nucleotide sequence encoding a polypeptide which has at least 95% identity to the amino acid sequence of SEQ ID NO:2, over the entire length of SEQ ID NO:2. In this regard, polypeptides which have at least 97% identity are highly preferred, whilst those with at least 98-99% identity are more highly preferred, and those with at least 99% identity are most highly preferred. Such polynucleotides include a polynucleotide comprising the nucleotide sequence contained in SEQ ID NO:1 encoding the polypeptide of SEQ ID NO:2.

Further polynucleotides of the present invention include isolated polynucleotides comprising a nucleotide sequence that has at least95% identity to a nucleotide sequence encoding a polypeptide of SEQ ID NO:2, over the entire coding region. In this regard, polynucleotides which have at least 97% identity are highly preferred, whilst those with at least 98-99% identity are more highly preferred, and those with at least 99% identity are most highly preferred.

Further polynucleotides of the present invention include isolated polynucleotides comprising a nucleotide sequence which has at least 95% identity to SEQ ID NO: 1 over the entire length of SEQ ID NO:1. In this regard, polynucleotides which have at least 97% identity are highly preferred, whilst those with at least 98-99% identiy are more highly preferred, and those with at least 99% identity are most highly preferred. Such polynucleotides include a polynucleotide comprising the polynucleotide of SEQ ID NO:1 as well as the polynucleotide of SEQ ID NO:1.

The invention also provides polynucleotides which are complementary to all the above described polynucleotides.

The nucleotide sequence of SEQ ID NO:1, disclosed in WO99/37762 (SmithKline Beecham) is a human cDNA sequence and comprises a polypeptide encoding sequence (nucleotide 9 to 1241) encoding a polypeptide of 411 amino acids, the polypeptide of SEQ ID NO:2. The nucleotide sequence encoding the polypeptide of SEQ ID NO:2 may be identical to the polypeptide encoding sequence contained in SEQ ID NO: 1 or it may be a sequence other than the one contained in SEQ ID NO:1, which, as a result of the redundancy (degeneracy) of the genetic code, also encodes the polypeptide of SEQ ID NO:2.

Preferred polypeptides and polynucleotides of the present invention are expected to have, *inter alia*, similar biological functions/properties to their homologous polypeptides and polynucleotides. Furthermore, preferred polypeptides and polynucleotides of the present invention have at least one h-TREK1 activity.

Polynucleotides of the present invention may be obtained, using standard cloning and screening techniques (Sambrook *et al*., Molecular Cloning: A Laboratory Manual, 2nd Ed., Cold Spring Harbor Laboratory Press, Cold Spring Harbor, N.Y.(1989)), from a cDNA library derived from mRNA in cells of human foetal brain, adrenal gland and ovary tumour. Polynucleotides of the invention can also be obtained from natural sources such as genomic DNA libraries or can be synthesized using well known and commercially available techniques.

When polynucleotides of the present invention are used for the recombinant production of polypeptides of the present invention, the polynucleotide may include the coding sequence for the mature polypeptide, by itself; or the coding sequence for the mature polypeptide in reading frame with other coding sequences, such as those encoding a leader or secretory sequence, a pre-, or pro- or prepro- protein sequence, or other fusion peptide portions. For example, a marker sequence which facilitates purification of the fused polypeptide can be encoded. In certain preferred embodiments of this aspect of the invention, the marker sequence is a hexa-histidine peptide, as provided in the pQE vector (Qiagen, Inc.) and described in Gentz *et al*., Proc Natl Acad Sci USA (1989) 86:821-824, or is an HA tag. The polynucleotide may also contain non-coding 5' and 3' sequences, such as transcribed, non-translated sequences, splicing and polyadenylation signals, ribosome binding sites and sequences that stabilize mRNA.

Further embodiments of the present invention include polynucleotides encoding polypeptide variants which comprise the amino acid sequence of SEQ ID NO:2 and in which several, for instance from 5 to 10, 1 to 5, 1 to 3, 1 to 2 or 1, amino acid residues are substituted, deleted or added, in any combination.

Recombinant polypeptides of the present invention may be prepared by processes well known in the art from genetically engineered host cells comprising expression systems. Accordingly, in a further aspect, the present invention relates to expression systems which comprise a polynucleotide or polynucleotides of the present invention, to host cells which are genetically engineered with such expression sytems and to the production of polypeptides of the invention by recombinant techniques. Cell-free translation systems can also be employed to produce such proteins using RNAs derived from the DNA constructs of the present invention.

For recombinant production, host cells can be genetically engineered to incorporate expression systems or portions thereof for polynucleotides of the present invention. Introduction of polynucleotides into host cells can be effected by methods described in many standard laboratory manuals, such as Davis et al, Basic Methods in Molecular Biology (1986) and Sambrook *et al*., Molecular Cloning: A Laboratory Manual, 2nd Ed., Cold Spring Harbor Laboratory Press, Cold Spring Harbor, N.Y. (1989). Preferred such methods include, for instance, calcium phosphate transfection, DEAE-dextran mediated transfection, microinjection, cationic lipid-mediated transfection, electroporation, transduction, scrape loading, ballistic introduction or infection.

Representative examples of appropriate hosts include bacterial cells, such as *Streptococci, Staphylococci, E. coli, Streptomyces* and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, HEK 293 and Bowes melanoma cells; and plant cells.

A great variety of expression systems can be used, for instance, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression systems may contain control regions that regulate as well as engender expression. Generally, any system or vector which is able to maintain, propagate or express a polynucleotide to produce a polypeptide in a host may be used. The appropriate nucleotide sequence may be inserted into an expression system by any of a variety of well-known and routine techniques, such as, for example, those set forth in Sambrook *et al*., Molecular Cloning, A Laboratory Manual (supra). Appropriate secretion signals may be incorporated into the desired polypeptide to allow secretion of the translated protein into the lumen of the endoplasmic reticulum, the periplasmic space or the extracellular environment. These signals may be endogenous to the polypeptide or they may be heterologous signals.

Polypeptides of the present invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding proteins may be employed to regenerate active conformation when the polypeptide is denatured during isolation and or purification.

The polypeptides of the invention or their fragments or analogs thereof, or cells expressing them, can also be used as immunogens to produce antibodies immunospecific for polypeptides of the present invention. The term " immunospecific" means that the antibodies have substantially greater affinity for the polypeptides of the invention than their affinity for other related polypeptides in the prior art.

Antibodies generated against polypeptides of the present invention may be obtained by administering the polypeptides or epitope-bearing fragments, analogs or cells to an animal, preferably a non-human animal, using routine protocols. For preparation of monoclonal antibodies, any technique which provides antibodies produced by continuous cell line cultures can be used. Examples include the hybridoma technique (Kohler, G. and Milstein, C., Nature (1975) 256:495-497), the trioma technique, the human B-cell hybridoma technique (Kozbor *et al*., Immunology Today (1983) 4:72) and the EBV-hybridoma technique (Cole *et al*., Monoclonal Antibodies and Cancer Therapy, 77-96, Alan R. Liss, Inc., 1985).

Techniques for the production of single chain antibodies, such as those described in U.S. Patent No. 4,946,778, can also be adapted to produce single chain antibodies to polypeptides of this invention. Also, transgenic mice, or other organisms, including other mammals, may be used to express humanized antibodies.

Antibodies against polypeptides of the present invention may also be employed to treat the Diseases, amongst others.

In a further aspect, the present invention relates to genetically engineered soluble fusion proteins comprising a polypeptide of the present invention, or a fragment thereof, and various portions of the constant regions of heavy or light chains of immunoglobulins of various subclasses (IgG, IgM, IgA, IgE). Preferred as an immunoglobulin is the constant part of the heavy chain of human IgG, particularly IgG1, where fusion takes place at the hinge region. In a particular embodiment, the Fc part can be removed simply by incorporation of a cleavage sequence which can be cleaved with blood clotting factor Xa. Furthermore, this invention relates to processes for the preparation of these fusion proteins by genetic engineering, and to the use thereof for drug screening, diagnosis and therapy. A further aspect of the invention also relates to polynucleotides encoding such fusion proteins. Examples of fusion protein technology can be found in International Patent Application Nos. WO94/29458 and WO94/22914.

Another aspect of the invention relates to the induction of an immunological response in a mammal inoculated with a polypeptide of the present invention, adequate to produce antibody and/or T cell immune response to protect said animal from the Diseases hereinbefore mentioned, amongst others. Yet another aspect of the invention relates to the induction of an immunological response in a mammal delivered a polypeptide of the present invention *via* a vector directing expression of the polynucleotide and coding for the polypeptide *in vivo* in order to induce such an immunological response to produce antibody to protect said animal from diseases.

The invention provides for an immunological/vaccine formulation (composition) which, when introduced into a mammalian host, induces an immunological response in that mammal to a polypeptide of the present invention wherein the composition comprises a polypeptide or polynucleotide of the present invention The vaccine formulation may further comprise a suitable carrier. Since a polypeptide may be broken down in the stomach, it is preferably administered parenterally (for instance, subcutaneous, intramuscular, intravenous, or intradermal injection). Formulations suitable for parenteral administration include aqueous and non-aqueous sterile injection solutions which may contain anti-oxidants, buffers, bacteriostats and solutes which render the formulation isotonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

Polypeptides of the present invention are responsible for one or more biological functions, including one or more disease states, in particular the Diseases hereinbefore mentioned. It is therefore desirous to devise screening methods to identify compounds which stimulate or which inhibit the function of the polypeptide. Accordingly, in a further aspect, the present invention provides for a method of screening compounds to identify those which stimulate or which inhibit the function of the polypeptide. In general, agonists or antagonists may be employed for therapeutic and prophylactic purposes for such Diseases as hereinbefore mentioned. Compounds may be identified from a variety of sources, for example, cells, cell-free preparations, chemical libraries, and natural product mixtures. Such agonists, antagonists or inhibitors so-identified may be natural or modified substrates, ligands, receptors, enzymes, etc., as the case may be, of the polypeptide; or may be structural or functional mimetics thereof (see Coligan *et al*., Current Protocols in Immunology 1(2):Chapter 5 (1991)).

The screening method may simply measure the binding of a candidate compound to the polypeptide, or to cells or membranes bearing the polypeptide, or a fusion protein thereof by means of a label directly or indirectly associated with the candidate compound. Alternatively, the screening method may involve competition with a labeled competitor. Further, these screening methods may test whether the candidate compound results in a signal generated by activation or inhibition of the polypeptide, using detection systems appropriate to the cells bearing the polypeptide. Inhibitors of activation are generally assayed in the presence of a known agonist and the effect on activation by the agonist by the presence of the candidate compound is observed. Constitutively active polpypeptides may be employed in screening methods for inverse agonists or inhibitors, in the absence of an agonist or inhibitor, by testing whether the candidate compound results in inhibition of activation of the polypeptide. Further, the screening methods may simply comprise the steps of mixing a candidate compound with a solution containing a polypeptide of the present invention, to form a mixture, measuring h-TREK1 activity in the mixture, and comparing the h-TREK1 activity of the mixture to a standard. Fusion proteins, such as those made from Fc portion and h-TREK I polypeptide, as hereinbefore described, can also be used for high-throughput screening assays to identify antagonists for the polypeptide of the present invention (see D. Bennett *et al*., J Mol Recognition, 8:52-58 (1995); and K. Johanson *et al*., J Biol Chem, 270(16):9459-9471 (1995)).

The polynucleotides, polypeptides and antibodies to the polypeptide of the present invention may also be used to configure screening methods for detecting the effect of added compounds on the production ofmRNA and polypeptide in cells. For example, an ELISA assay may be constructed for measuring secreted or cell associated levels of polypeptide using monoclonal and polyclonal antibodies by standard methods known in the art. This can be used to discover agents which may inhibit or enhance the production of polypeptide (also called antagonist or agonist, respectively) from suitably manipulated cells or tissues.

The polypeptide may be used to identify interacting proteins or other molecules. For example the identification of interacting kinases may help to elucidate the signalling pathway of which h-TREK1 forms a part. These methods include, but are not limited to, two-hybrid system (Fields and Song, Nature 340, pp. 245-246 (1989); Durfee et al., Genes Dev. 7, pp. 555-569 (1993); Bartel and Fields, Methods in Enzymology 254, pp. 241-262 (1995)), λgt11 expression cloning (Blackwood and Eisenmann, Methods in Enzymology 254, pp. 229-240 (1995)), expression screening for protein kinase substrates (Fukunaga and Hunter, EMBO J. 16, pp. 1921-1933 (1997)) as well as coimmunoprecipitation and western blotting assays (Ransone, Methods in Enzymology 254, pp. 491-496 (1995), Okamura et al., Methods in Enzymology 254, pp. 535-549 (1995)). These screening methods may also be used to identify.agonists and antagonists of the polypeptide which compete with the binding of the polypeptideto its receptors, if any. Standard methods for conducting such assays are well understood in the art.

Examples of potential polypeptide antagonists include antibodies or, in some cases, oligonucleotides or proteins which are closely related to the ligands, substrates, receptors, enzymes, etc., as the case may be, of the polypeptide, e.g., a fragment of the ligands, substrates, receptors, enzymes, etc.; or small molecules which bind to the polypetide of the present invention but do not elicit a response, so that the activity of the polypeptide is prevented.

The present invention describes a screening kit for identifying agonists, antagonists, ligands, receptors, substrates, enzymes, etc. for polypeptides of the present invention; or compounds which decrease or enhance the production of such polypeptides, which comprises:

(a) a polypeptide of the present invention;
(b) a recombinant cell expressing a polypeptide of the present invention;
(c) a cell membrane expressing a polypeptide of the present invention; or
(d) antibody to a polypeptide of the present invention; which polypeptide is preferably that of SEQ ID NO:2.

It will be appreciated that in any such kit, (a), (b), (c) or (d) may comprise a substantial component.

It will be readily appreciated by the skilled artisan that a polypeptide of the present invention may also be used in a method for the structure-based design of an agonist, antagonist or inhibitor of the polypeptide, by:

(a) determining in the first instance the three-dimensional structure of the polypeptide;
(b) deducing the three-dimensional structure for the likely reactive or binding site(s) of an agonist, antagonist or inhibitor;
(c) synthesing candidate compounds that are predicted to bind to or react with the deduced binding or reactive site; and
(d) testing whether the candidate compounds are indeed agonists, antagonists or inhibitors. It will be further appreciated that this will normally be an interative process.

The present invention provides for the treatment of abnormal conditions i.e. epilepsy, sleep-related disorders, the induction of sleep, cognitive dysfunction/enhancement, attention deficit disorder, addiction, anxiety / phobia, dyskinesias including Parkinson's and Huntington's chorea. Cerebral palsy, incontinence, erectile dysfunction, alopecia, related to either an excess of, or an under-expression of, h-TREK1 polypeptide activity.

If the activity of the polypeptide is in excess, several approaches are available. One approach comprises administering to a subject in need thereof an inhibitor compound (antagonist) as hereinabove described, optionally in combination with a pharmaceutically acceptable carrier, in an amount effective to inhibit the function of the polypeptide, such as, for example, by blocking the binding of ligands, substrates, receptors, enzymes, etc., or by inhibiting a second signal, and thereby alleviating the abnormal condition. In another approach, soluble forms of the polypeptides still capable of binding the ligand, substrate, enzymes, receptors, etc. in competition with endogenous polypeptidemay be administered. Typical examples of such competitors include fragments of the h-TREK1 polypeptide.

In still another approach, expression of the gene encoding endogenous h-TREK1 polypeptide can be inhibited using expression blocking techniques. Known such techniques involve the use of antisense sequences, either internally generated or externally administered (see, for example, O'Connor, J Neurochem (1991) 56:560 in Oligodeoxynucleotides as Antisense Inhibitors of Gene Expression, CRC Press, Boca Raton, FL (1988)). Alternatively, oligonucleotides which form triple helices ("triplexes") with the gene can be supplied (see, for example, Lee *et al*., Nucleic Acids Res (1979) 6:3073; Cooney *et al*., Science (1988) 241:456; Dervan *et al*., Science (1991) 251:1360). These oligomers can be administered *per se* or the relevant oligomers can be expressed *in vivo*. Synthetic antisense or triplex oligonucleotides may comprise modified bases or modified backbones. Examples of the latter include methylphosphonate, phosphorothioate or peptide nucleic acid backbones. Such backbones are incorporated in the antisense or triplex oligonucleotide in order to provide protection from degradation by nucleases and are well known in the art. Antisense and triplex molecules synthesised with these or other modified backbones also form part of the present invention.

In addition, expression of the human h-TREK1 polypeptide may be prevented by using ribozymes specific to the human h-TREK1 mRNA sequence. Ribozymes are catalytically active RNAs that can be natural or synthetic (see for example Usman, N, et al., Curr. Opin. Struct. Biol (1996) 6(4), 527-33.). Synthetic ribozymes can be designed to specifically cleave human h-TREK1 mRNAs at selected positions thereby preventing translation of the human h-TREK1 mRNAs into functional polypeptide. Ribozymes may be synthesised with a natural ribose phosphate backbone and natural bases, as normally found in RNA molecules. Alternatively the ribosymes may be synthesised with non-natural backbones to provide protection from ribonuclease degradation, for example, 2'-O-methyl RNA, and may contain modified bases.

For treating abnormal conditions related to an under-expression of h-TREK and its activity, several approaches are also available. One approach comprises administering to a subject a therapeutically effective amount of a compound which activates a polypeptide of the present invention, i.e., an agonist as described above, in combination with a pharmaceutically acceptable carrier, to thereby alleviate the abnormal condition. Alternatively, gene therapy may be employed to effect the endogenous production of h-TREK1 by the relevant cells in the subject. For example, a polynucleotide of the invention may be engineered for expression in a replication defective retroviral vector, as discussed above. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a subject for engineering cells *in vivo* and expression of the polypeptide *in vivo.* For an overview of gene therapy, see Chapter 20, Gene Therapy and other Molecular Genetic-based Therapeutic Approaches, (and references cited therein) in Human Molecular Genetics, T Strachan and A P Read, BIOS Scientific Publishers Ltd (1996). Another approach is to administer a therapeutic amount of a polypeptide of the present invention in combination with a suitable pharmaceutical carrier.

The present invention describes pharmaceutical compositions comprising a therapeutically effective amount of a polypeptide, such as the soluble form of a polypeptide of the present invention, agonist/antagonist peptide or small molecule compound, in combination with a pharmaceutically acceptable carrier or excipient. Such carriers include, but are not limited to, saline, buffered saline, dextrose, water, glycerol, ethanol, and combinations thereof. The invention further relates to pharmaceutical packs and kits comprising one or more containers filled with one or more of the ingredients of the aforementioned compositions of the invention. Polypeptides and other compounds of the present invention may be employed alone or in conjunction with other compounds, such as therapeutic compounds.

The composition will be adapted to the route of administration, for instance by a systemic or an oral route. Preferred forms of systemic administration include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if a polypeptide or other compounds of the present invention can be formulated in an enteric or an encapsulated formulation, oral administration may also be possible. Administration of these compounds may also be topical and/or localized, in the form of salves, pastes, gels, and the like.

The dosage range required depends on the choice of peptide or other compounds of the present invention, the route of administration, the nature of the formulation, the nature of the subject's condition, and the judgment of the attending practitioner. Suitable dosages, however, are in the range of 0.1-100 μg/kg of subject. Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art.

Polypeptides used in treatment can also be generated endogenously in the subject, in treatment modalities often referred to as" gene therapy" as described above. Thus, for example, cells from a subject may be engineered with a polynucleotide, such as a DNA or RNA, to encode a polypeptide *ex vivo*, and for example, by the use of a retroviral plasmid vector. The cells are then introduced into the subject.

The following definitions are provided to facilitate understanding of certain terms used frequently hereinbefore.

" Antibodies" as used herein includes polyclonal and monoclonal antibodies, chimeric, single chain, and humanized antibodies, as well as Fab fragments, including the products of an Fab or other immunoglobulin expression library.

" Isolated" means altered " by the hand of man" from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide separated from the coexisting materials of its natural state is " isolated", as the term is employed herein.

" Polynucleotide" generally refers to any polyribonucleotide or polydeoxribonucleotide, which may be unmodified RNA or DNA or modified RNA or DNA. " Polynucleotides" include, without limitation, single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term "polynucleotide" also includes DNAs or RNAs containing one or more modified bases and DNAs or RNAs with backbones modified for stability or for other reasons. " Modified" bases include, for example, tritylated bases and unusual bases such as inosine. A variety of modifications may be made to DNA and RNA; thus, "polynucleotide" embraces chemically, enzymatically or metabolically modified forms of polynucleotides as typically found in nature, as well as the chemical forms of DNA and RNA characteristic of viruses and cells. " Polynucleotide" also embraces relatively short polynucleotides, often referred to as oligonucleotides.

" Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. " Polypeptides" include amino acid sequences modified either by natural processes, such as post-translational processing, or by chemical modification techniques which are well known in the art. Such modifications are well described in basic texts and in more detailed monographs, as well as in a voluminous research literature. Modifications may occur anywhere in a polypeptide, including the peptide backbone, the amino acid side-chains and the amino or carboxyl termini. It will be appreciated that the same type of modification may be present to the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from post-translation natural processes or may be made by synthetic methods. Modifications include acetylation, acylation, ADP-ribosylation, amidation, biotinylation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination (see, for instance, Proteins - Structure and Molecular Properties, 2nd Ed., T. E. Creighton, W. H. Freeman and Company, New York, 1993; Wold, F., Post-translational Protein Modifications: Perspectives and Prospects, pgs. 1-12 in Post-translational Covalent Modification of Proteins, B. C. Johnson, Ed., Academic Press, New York, 1983; Seifter *et al*., "Analysis for protein modifications and nonprotein cofactors", Meth Enzymol (1990) 182: 626-646 and Rattan *et al*., "Protein Synthesis: Post-translational Modifications and Aging", Ann NY Acad Sci (1992) 663:48-62). .

"Variant" refers to a polynucleotide or polypeptide that differs from a reference polynucleotide or polypeptide, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

"Identity," as known in the art, is a relationship between two or more polypeptide sequences or two or more polynucleotide sequences, as determined by comparing the sequences. In the art, "identity" also means the degree of sequence relatedness between polypeptide or polynucleotide sequences, as the case may be, as determined by the match between strings of such sequences. "Identity" and "similarity" can be readily calculated by known methods, including but not limited to those described in (*Computational Molecular Biology,* Lesk, A.M., ed., Oxford University Press, New York, 1988; *Biocomputing: Informatics and Genome Projects,* Smith, D.W., ed., Academic Press, New York, 1993; *Computer Analysis of Sequence Data*, Part I, Griffin, A.M., and Griffin, H.G., eds., Humana Press, New Jersey, 1994; *Sequence Analysis in Molecular Biology,* von Heinje, G., Academic Press, 1987; and *Sequence Analysis Primer,* Gribskov, M. and Devereux, J., eds., M Stockton Press, New York, 1991; and Carillo, H., and Lipman, D., SIAM *J. Applied Math., 48:* 1073 (1988). Preferred methods to determine identity are designed to give the largest match between the sequences tested. Methods to determine identity and similarity are codified in publicly available computer programs. Preferred computer program methods to determine identity and similarity between two sequences include, but are not limited to, the GCG program package (Devereux, J., et al., *Nucleic Acids Research 12(1):* 387 (1984)), BLASTP, BLASTN, and FASTA (Atschul, S.F. et al., *J. Molec. Biol. 215:* 403-410 (1990). The BLAST X program is publicly available from NCBI and other sources (*BLAST Manual*, Altschul, S., *et al*., NCBI NLM NIH Bethesda, MD 20894; Altschul, S., *et al., J. Mol. Biol. 215*: 403-410 (1990). The well known Smith Waterman algorithm may also be used to determine identity.

Preferred parameters for polypeptide sequence comparison include the following:

1) Algorithm: Needleman and Wunsch, J. Mol Biol. 48: 443-453 (1970)
Comparison matrix: BLOSSUM62 from Hentikoff and Hentikoff, Proc. Natl. Acad. Sci. USA. 89:10915-10919 (1992)
Gap Penalty: 12
Gap Length Penalty: 4

A program useful with these parameters is publicly available as the "gap" program from Genetics Computer Group, Madison WI. The aforementioned parameters are the default parameters for peptide comparisons (along with no penalty for end gaps).

Preferred parameters for polynucleotide comparison include the following:

1) Algorithm: Needleman and Wunsch, J. Mol Biol. 48: 443-453 (1970)
Comparison matrix: matches = +10, mismatch = 0
Gap Penalty: 50

Gap Length Penalty: 3
Available as: The "gap" program from Genetics Computer Group, Madison WI. These are the default parameters for nucleic acid comparisons.

By way of example, a polynucleotide sequence of the present invention may be identical to the reference sequence of SEQ ID NO: 1, that is be 100% identical, or it may include up to a certain integer number of nucleotide alterations as compared to the reference sequence. Such alterations are selected from the group consisting of at least one nucleotide deletion, substitution, including transition and transversion, or insertion, and wherein said alterations may occur at the 5' or 3' terminal positions of the reference nucleotide sequence or anywhere between those terminal positions, interspersed either individually among the nucleotides in the reference sequence or in one or more contiguous groups within the reference sequence. The number of nucleotide alterations is determined by multiplying the total number of nucleotides in SEQ ID NO: I by the numerical percent of the respective percent identity(divided by 100) and subtracting that product from said total number of nucleotides in SEQ ID NO:1, or:

$$n_n \leq x_n - (x_n \bullet y),$$

wherein $\mathbf{n}_n$ is the number of nucleotide alterations, $\mathbf{x}_n$ is the total number of nucleotides in SEQ ID NO:1, and **y** is, for instance, 0.70 for 70%, 0.80 for 80%, 0.85 for 85%, 0.90 for 90%, 0.95 for 95%,etc., and wherein any non-integer product of $\mathbf{x}_n$ and **y** is rounded down to the nearest integer prior to subtracting it from $\mathbf{x}_n$. Alterations of a polynucleotide sequence encoding the polypeptide of SEQ ID NO:2 may create nonsense, missense or frameshift mutations in this coding sequence and thereby alter the polypeptide encoded by the polynucleotide following such alterations.

Similarly, a polypeptide sequence of the present invention may be identical to the reference sequence of SEQ ID NO:2, that is be 100% identical, or it may include up to a certain integer number of amino acid alterations as compared to the reference sequence such that the % identity is less than 100%. Such alterations are selected from the group consisting of at least one amino acid deletion, substitution, including conservative and non-conservative substitution, or insertion, and wherein said alterations may occur at the amino- or carboxy-terminal positions of the reference polypeptide sequence or anywhere between those terminal positions, interspersed either individually among the amino acids in the reference sequence or in one or more contiguous groups within the reference sequence. The number of amino acid alterations for a given % identity is determined by multiplying the total number of amino acids in SEQ ID NO:2 by the numerical percent of the respective percent identity(divided by 100) and then subtracting that product from said total number of amino acids in SEQ ID NO:2, or:

$$n_a \leq x_a - (x_a \bullet y),$$

wherein $\mathbf{n}_a$ is the number of amino acid alterations, $\mathbf{x}_a$ is the total number of amino acids in SEQ ID NO:2, and **y** is, for instance 0.70 for 70%, 0.80 for 80%, 0.85 for 85% etc., and wherein any non-integer product of $\mathbf{x}_a$ and **y** is rounded down to the nearest integer prior to subtracting it from $\mathbf{x}_a$.

"Homolog" is a generic term used in the art to indicate a polynucleotide or polypeptide sequence possessing a high degree of sequence relatedness to a subject sequence. Such relatedness may be quantified by determining the degree of identity and/or similarity between the sequences being compared as hereinbefore described. Falling within this generic term are the terms "ortholog", meaning a polynucleotide or polypeptide that is the functional equivalent of a polynucleotide or polypeptide in another species, and "paralog" meaning a functionally similar sequence when considered within the same species.

"Fusion protein" refers to a protein encoded by two, often unrelated, fused genes or fragments thereof. In one example, EP-A-0 464 discloses fusion proteins comprising various portions of constant region of immunoglobulin molecules together with another human protein or part thereof. In many cases, employing an immunoglobulin Fc region as a part of a fusion protein is advantageous for use in therapy and diagnosis resulting in, for example, improved pharmacokinetic properties [see, e.g., EP-A 0232 262]. On the other hand, for some uses it would be desirable to be able to delete the Fc part after the fusion protein has been expressed, detected and purified.

**Example - Tissue localisation**

***Materials***

*Animals*

Adult male Wistar rats (200-250 g, Charles River) were kept in a fixed 12 hour light-dark cycle with food and water provided *ad libitum.*

***Methods***

*Production of synthetic peptides and antibodies*

A synthetic peptide corresponding to amino-acids 5-21 from the intracellular N-terminal domain of mouse (Fink et al. (1996) EMBO Journal 15, 6854-6862) and human TREK-1 (Meadows et al.,(2000) Pflugers Arch. 439, 714-722) (DLLDPKSAAQNSKPRLS) was synthesised, with an additional cysteine at the N-terminal end (Immune Systems Ltd (ISL), Paignton, UK). This sequence was confirmed to be identical to the corresponding sequence in rat TREK-1. This peptide sequence was not found in any of the other protein sequences filed in GenEMBL or SWISSPROT (BLAST, peptide sequence against nucleotide/peptide databases, Advanced search). The synthesised peptide was conjugated to keyhole limpet hemocyanin and injected into rabbits for the production of polyclonal antisera. For affinity purification the peptide was conjugated to an activated sepharose support and the polysera passed through the column (ISL).

*Immunohistochemistry (IHC)*

Immunosignals were revealed with an ABC peroxidase reporter system as previously described (Hervieu and Emson (1998) Neuroscience 85, 1263-1284) or by immunofluorescence. Adult male Sprague-Dawley rats were transcardiacally perfused with 4% paraformaldehyde (PFA) in phosphate buffered saline (PBS) after receiving an overdose of pentobarbitone in accordance with U.K. Home Office regulations and under provision of the appropriate animal licence. Following sacrifice the brains were removed, post-fixed in 4% paraformaldehyde in PBS for 2 hours at 4°C and transferred into a 20% sucrose/PBS solution overnight. Following freezing of the brains in isopentane, 35μm coronal sections were cut in a cryostat. Sections were stored in 0.5% PFA in PBS at 4°C. Sections were incubated in PBS containing 20% methanol and 1.5% hydrogen peroxide for 30 minutes in order to quench the endogenous peroxidase activity. Following two five-minute washes in PBS, sections were placed in a blocking solution (3% normal goat serum, 2g/l bovine serum albumin, 0.1% Triton X100 in PBS) for 30-45 minutes. Sections were then incubated with the primary TREK-1 antiserum at 1/10 dilution for 48 hours at 4°C with gentle agitation. After primary incubation, sections were given three ten-minute washes in 0.3% Triton X100 in PBS and then processed for peroxidase immunostaining using the Vector ABC (avidin:biotinylated-enzyme complex) (Vector Laboratories, Peterborough, UK) amplification system following the manufacturer's recommendations. Sections were incubated for one hour at room temperature in Vector goat anti-rabbit IgG (H + L) (Vector Laboratories) at 1 drop per 10ml blocking solution with gentle shaking, then given three ten-minute washes in 0.3% Triton X100 in PBS, and incubated under the same conditions in ABC solution at 2 drops per 10ml PBS which was prepared at least 30 minutes prior to use. After a further three ten-minute washes in 0.3% Triton X100 in PBS, sections were transferred into tris-buffered saline (TBS). Sections were incubated in 3,3'-diaminobenzidine (DAB) substrate (Vector Laboratories) for 5-10 minutes before the reaction was stopped in distilled water. Sections were mounted onto Superfrost polished slides (BDH), allowed to airdry, then coverslipped with DPX mountant.

Immunochemical fluorescence was carried out as previously described (Hervieu and Nahon (1995) Neuroendocrinology 61, 348-364) using one of several different streptavidin-conjugated fluorophores (Cy2, Cy3, Cy5, Red-Phycoerythrin, FITC, TRITC, AMCA; all from Jackson Laboratories).

Further sections were processed using fluorescent secondary antibodies for double labelling of TREK-1 and GABA. Sections were incubated in blocking solution (as described hereinabove) for 30-45 minutes before simultaneous incubation overnight at 4°C with TREK-1 antiserum used at a dilution of 1/10 and Guinea pig anti-GABA polyclonal antibody (Chemicon) used at a dilution of 1/1000. After three ten minute washes in 0.3% TritonX 100 in PBS, all sections were incubated for one hour at room temperature in blocking solution containing goat anti-guinea pig biotinylated antibody (Vector; one drop per 10ml) and Cy2-conjugated AffiniPure F(ab')2 fragment goat anti-rabbit IgG (1/100). Sections were washed as before then incubated in Cy-5 conjugated Streptavidin (1/900) for one hour at room temperature. Sections were kept in the dark during and after incubation with fluorescent antibodies. The tissue was given three final ten minute washes in 0.3% TritonX 100 in PBS then mounted and coverslipped with Citifluor.

Specificity of the antiserum was assessed by preabsorption with the peptide it was designed against. The antiserum was incubated at working dilution either alone or together with 1µM or 10µM peptide overnight at 4°C. This solution was then used as the primary antibody following the chromogenic immunohistochemistry protocol outlined above.

## RESULTS

Table 1 shows the relative densities of TREK-1 immunosignals in the rat brain and spinal cord. The relative density of labelling is classified as:
absent (-) sparse (+) moderate (++) extensive (+++)

| **Region Telencephalon** | **Immunosignals (cell bodies and processes)** |
|---|---|
| *Olfactory system* | |
| Dorsal endopiriform nucleus | + |
| Islands of Calleja | ++ |
| Olfactory nuclei | ++ |
| Piriform cortex | +++ |
| Tenia tecta | ++ |
| *Neocortex* | |
| Agranular insular cortex | ++ |
| Frontal cortex | +++ |
| Granular insular cortex | + |
| Parietal cortex | +++ |
| *Metacortex* | |
| Cingulate/Retrosplenial cortex | +++ |
| *Basal Ganglia* | |
| Caudate putamen | + |
| Substantia nigra | ++ |
| Globus pallidus | + |
| Subthalamic nucleus | +++ |
| *Hippocampal formation* | |
| CA1 region | + |
| CA2 region | +++ |
| CA3 region | + |
| Dentate gyrus | +++ |
| Subiculum | +++ |
| *Amygdala* | |
| Amygdaloid nuclei | + |
| Substantia innominata | ++ |
| *Septal and basal magnocellular nuclei* | |
| Accumbens nucleus | - |
| Bed nucleus of the stria terminalis | ++ |
| Lateral septal nucleus, dorsal part | ++ |
| Medial septal nucleus | +++ |
| Nucleus of the horizontal limb of the diagonal band | +++ |

(continued)

| Region Telencephalon | Immunosignals (cell bodies and processes) |
|---|---|
| **Diencephalon** | |
| *Thalamus* | |
| Anteroventral thalamic nucleus | + |
| Centrolateral thalamic nucleus | + |
| Centromedial thalamic nucleus | + |
| Geniculate nuclei | + |
| Lateral habenular nucleus | +++ |
| Medial habenular nucleus | - |
| Parafascicular thalamic nucleus | +++ |
| Paraventricular thalamic nucleus | - |
| Reticularthalamic nucleus | +++ |
| Ventral posterolateral thalamic nucleus | ++ |
| Ventral posteromedial thalamic nucleus | +++ |
| Ventrolateral thalamic nucleus | ++ |
| Ventromedial thalamic nucleus | + |
| Zona incerta | +++ |
| *Hypothalamus* | |
| Anterior hypothalamic area | ++ |
| Arcuate hypothalamic nucleus | ++ |
| Lateral hypothalamic area (LHA) | ++ |
| Lateral mammillary nucleus | +++ |
| Medial mammillary nucleus | ++ |
| Paraventricular hypothalamic nucleus | +++ |
| Posterior hypothalamic area | ++ |
| **Mesencephalon** | |
| Anterior pretectal nucleus | ++ |
| Dorsal tegmental nucleus | +++ |
| Inferior colliculus | +++ |
| Interpeduncular nuclei | +++ |
| Oculomotor nucleus | +++ |
| Periaqueductal grey | +++ |
| Principal sensory trigeminal nucleus | +++ |
| Raphe nuclei | ++ |
| Red nucleus | +++ |
| Substantia nigra | +++ |
| Superior colliculus | ++ |
| Ventral tegmental area | ++ |
| **Rhombencephalon** | |
| Cochlear nucleus complex | ++ |
| Facial nucleus | +++ |
| Parabrachial nuclei | ++ |
| Sublocus coeruleus | + |
| Olivary complex | +++ |
| Pontine reticular nucleus | ++ |
| Spinal trigeminal nucleus | +++ |
| Vestibular nucleus | +++ |

(continued)

| Cerebellum | |
|---|---|
| Cerebellar cortex | +++ |
| **Spinal cord** | +++ |

**SEQUENCE INFORMATION**

**SEQ ID NO:1**

GAATAAGAATGGCGGCACCTGACTTGCTGGATCCTAAATCTGCCGCTCAGAACTCCAAAC
CGAGGCTCTCGTTTTCCACGAAACCCACAGTGCTTGCTTCCCGGGTGGAGAGTGACACGA
CCATTAATGTTATGAAATGGAAGACGGTCTCCACGATATTCCTGGTGGTTGTCCTCTATC
TGATCATCGGAGCCACCGTGTTCAAAGCATTGGAGCAGCCTCATGAGATTTCACAGAGGA
CCACCATTGTGATCCAGAAGCAAACATTCATATCCCAACATTCCTGTGTCAATTCGACGG
AGCTGGATGAACTCATTCAGCAAATAGTGGCAGCAATAAATGCAGGGATTATACCGTTAG
GAAACACCTCCAATCAAATCAGTCACTGGGATTTGGGAAGTTCCTTCTTCTTTGCTGGCA
CTGTTATTACAACCATAGGATTTGGAAACATCTCACCACGCACAGAAGGCGGCAAAATAT
TCTGTATCATCTATGCCTTACTGGGAATTCCCCTCTTTGGTTTTCTCTTGGCTGGAGTTG
GAGATCAGCTAGGCACCATATTTGGAAAAGGAATTGCCAAAGTGGAAGATACGTTTATTA
AGTGGAATGTTAGTCAGACCAAGATTCGCATCATCTCAACAATCATATTTATACTATTTG
GCTGTGTACTCTTTGTGGCTCTGCCTGCGATCATATTCAAACACATAGAAGGCTGGAGTG
CCCTGGACGCCATTTATTTTGTGGTTATCACTCTAACAACTATTGGATTTGGTGACTACG
TTGCAGGTGGATCCGATATTGAATATCTGGACTTCTATAAGCCTGTCGTGTGGTTCTGGA
TCCTTGTAGGGCTTGCTTACTTTGCTGCTGTCCTGAGCATGATTGGAGATTGGCTCCGAG
TGATATCTAAAAAGACAAAAGAAGAGGTGGGAGAGTTCAGAGCACACGCTGCTGAGTGGA
CAGCCAACGTCACAGCCGAATTCAAAGAAACCAGGAGGCGACTGAGTGTGGAGATTTATG
ACAAGTTCCAGCGGGCCACCTCCATCAAGCGGAAGCTCTCGGCAGAACTGGCTGGAAACC
ACAATCAGGAGCTGACTCCTTGTAGGAGGACCCTGTCAGTGAACCACCTGACCAGCGAGA
GGGATGTCTTGCCTCCCTTACTGAAGACTGAGAGTATCTATCTGAATGGTTTGACGCCAC
ACTGTGCTGGTGAAGAGATTGCTGTGATTGAGAACATCAAATAGCC

**SEQ ID NO:2**

MAAPDLLDPKSAAQNSKPRLSFSTKPTVLASRVESDTTINVMKWKTVSTIFLVVVLYLII
GATVFKALEQPHEISQRTTIVIQKQTFISQHSCVNSTELDELIQQIVAAINAGIIPLGNT
SNQISHWDLGSSFFFAGTVITTIGFGNISPRTEGGKIFCIIYALLGIPLFGFLLAGVGDQ
LGTIFGKGIAKVEDTFIKWNVSQTKIRIISTIIFILFGCVLFVALPAIIFKHIEGWSALD
AIYFVVITLTTIGFGDYVAGGSDIEYLDFYKPVVWFWILVGLAYFAAVLSMIGDWLRVIS
KKTKEEVGEFRAHAAEWTANVTAEFKETRRRLSVEIYDKFQRATSIKRKLSAELAGNHNQ
ELTPCRRTLSVNHLTSERDVLPPLLKTESIYLNGLTPHCAGEEIAVIENIK

## Claims

1. The use of a compound selected from:

   (a) an h-TREK1 polypeptide;
   (b) a compound which activates an h-TREK1 polypeptide; or
   (c) a compound which inhibits an h-TREK1 polypeptide; or
   (d) a polynucleotide encoding an h-TREK1 polypeptide,

   for the manufacture of a medicament for treating:

   (i) epilepsy;
   (ii) sleep-related disorders;
   (iii) cognitive dysfunction;
   (iv) attention deficit disorder,
   (v) addiction;
   (vi) anxiety / phobia;
   (vii) dyskinesias including Parkinson's and Huntington's chorea;
   (viii) cerebral palsy;
   (ix) incontinence;
   (x) erectile dysfunction; or
   (xi) alopecia.

2. The use according to claim 1 wherein the medicament is used in the treatment of epilepsy.

3. The use according to claim 1 wherein the medicament is used in the treatment of sleep-related disorders.

4. The use according to claim 3 wherein the medicament is used to enhance the induction of sleep.

5. The use according to claim 1 wherein the medicament is used to enhance cognition or cognitive dysfunction.

6. The use according to claim 1 wherein the medicament is used in the treatment of dyskinesias including Parkinson's and Huntington's chorea

7. The use according to any one of claims 1 to 6 wherein the medicament comprises an isolated polypeptide which comprises a polypeptide having at least 95% identity to the h-TREK1 polypeptide of SEQ ID NO:2.

8. The use according to claim 7 wherein the isolated polypeptide is the h-TREK1 polypeptide of SEQ ID NO:2.

9. The use according to any one of claims 1 to 6 wherein the medicament comprises a compound which inhibits the activity of a h-TREK1 polypeptide.

10. The use according to any one of claims 1 to 6 wherein the medicament comprises a compound which activates an h-TREK1 polypeptide.

11. The use according to any one of claims 1 to 6 wherein the medicament comprises a polynucleotide encoding a polypeptide having at least 95% identity with the amino acid sequence of SEQ ID NO:2.

**12.** The use according to claim 11 wherein the polynucleotide comprises a polynucleotide having at least 95% identity with the polynucleotide of SEQ ID NO: 1.

**13.** The use according to claims 11 or 12 wherein the polynucleotide has the polynucleotide sequence of SEQ ID NO: 1.

**Patentansprüche**

**1.** Verwendung einer Verbindung, ausgewählt aus:

(a) einem h-TREK1-Polypeptid;
(b) einer Verbindung, die ein h-TREK1-Polypeptid aktiviert; oder
(c) einer Verbindung, die ein h-TREK1-Polypeptid inhibiert; oder
(d) einem Polynukleotid, kodierend ein h-TREKI-Polypeptid,

zur Herstellung eines Medikamentes zur Behandlung von:

(i) Epilepsie;
(ii) Schlafstörungen;
(iii) kognitiver Störung;
(iv) Aufmerksamkeitsdefizitstörung;
(v) Abhängigkeit;
(vi) Angst/Phobie;
(vii) Dyskinesien, einschließlich der Parkinsonschen und Huntingtonschen Erkrankung;
(viii) Zerebralparese
(ix) Inkontinenz
(x) Erektionsstörung; oder
(xi) Alopezie.

**2.** Verwendung nach Anspruch 1, worin das Medikament zur Behandlung von Epilepsie eingesetzt wird.

**3.** Verwendung nach Anspruch 1, worin das Medikament zur Behandlung von Schlafstörungen eingesetzt wird.

**4.** Verwendung nach Anspruch 3, worin das Medikament zur Förderung der Schlafinduktion eingesetzt wird.

**5.** Verwendung nach Anspruch 1, worin das Medikament zur (Ver)besserung der Kognition oder kognitiven Störung eingesetzt wird.

**6.** Verwendung nach Anspruch 1, worin das Medikament zur Behandlung von Dyskinesien, einschließlich der Parkinsonschen und Huntingtonschen Erkrankung eingesetzt wird.

**7.** Verwendung nach einem der Ansprüche 1 bis 6, worin das Medikament ein isoliertes Polypeptid umfasst, das ein Polypeptid mit mindestens 95%iger Identität mit dem h-TREK1-Polypeptid von SEQ ID NO:2 umfasst.

**8.** Verwendung nach Anspruch 7, worin das isolierte Polypeptid das h-TREK1-Polypeptid von SEQ ID NO:2 darstellt.

**9.** Verwendung nach einem der Ansprüche 1 bis 6, worin das Medikament eine Verbindung umfasst, welche die Aktivität eines h-TREK1-Polypeptids inhibiert.

**10.** Verwendung nach einem der Ansprüche 1 bis 6, worin das Medikament eine Verbindung umfasst, welche ein h-TREK1-Polypeptid aktiviert.

**11.** Verwendung nach einem der Ansprüche 1 bis 6, worin das Medikament ein Polynukleotid, kodierend ein Polypeptid mit mindestens 95%iger Identität mit der Aminosäuresequenz von SEQ ID NO:2 umfasst.

**12.** Verwendung nach Anspruch 11, worin das Polynukleotid ein Polynukleotid mit mindestens 95%iger Identität mit dem Polynukleotid von SEQ ID NO:1 umfasst.

13. Verwendung nach Anspruch 11 oder 12, worin das Polynukleotid die Polynukleotidsequenz von SEQ ID NO:1 aufweist.

## Revendications

1. Utilisation d'un composé choisi parmi :

   (a) un polypeptide h-TREK1 ;
   (b) un composé activant un polypeptide h-TREK1 ; ou
   (c) un composé inhibant un polypeptide h-TREK1 ; ou
   (d) un polynucléotide codant pour un polypeptide h-TREK1,

   pour la fabrication d'un médicament pour le traitement de :

   (i) l'épilepsie ;
   (ii) les troubles liés au sommeil ;
   (iii) le dysfonctionnement cognitif ;
   (iv) le trouble de l'attention ;
   (v) l'attachement maladif ;
   (vi) l'anxiété/la phobie ;
   (vii) les dyskinésies, y compris la chorée de Parkinson et de Huntington ;
   (viii) l'infirmité motrice cérébrale ;
   (ix) l'incontinence ;
   (x) le dysfonctionnement érectile ; ou
   (xi) l'alopécie.

2. Utilisation selon la revendication 1, dans laquelle le médicament est utilisé dans le traitement de l'épilepsie.

3. Utilisation selon la revendication 1, dans laquelle le médicament est utilisé dans le traitement des troubles liés au sommeil.

4. Utilisation selon la revendication 3, dans laquelle le médicament est utilisé pour améliorer l'induction du sommeil.

5. Utilisation selon la revendication 1, dans laquelle le médicament est utilisé pour améliorer la cognition ou le dysfonctionnement cognitif.

6. Utilisation selon la revendication 1, dans laquelle le médicament est utilisé dans le traitement des dyskinésies, y compris la chorée de Parkinson et de Huntington.

7. Utilisation selon l'une quelconque des revendications 1 à 6, dans laquelle le médicament comprend un polypeptide isolé comprenant un polypeptide ayant au moins 95% d'identité par rapport au polypeptide h-TREK1 de SEQ ID n°2.

8. Utilisation selon la revendication 7, dans laquelle le polypeptide isolé est le polypeptide h-TREK1 de SEQ ID n°2.

9. Utilisation selon l'une quelconque des revendications 1 à 6, dans laquelle le médicament comprend un composé inhibant l'activité d'un polypeptide h-TREK1.

10. Utilisation selon l'une quelconque des revendications 1 à 6, dans laquelle le médicament comprend un composé activant un polypeptide h-TREK1.

11. Utilisation selon l'une quelconque des revendications 1 à 6, dans laquelle le médicament comprend un polynucléotide codant pour un polypeptide ayant au moins 95% d'identité par rapport à la séquence d'acides aminés de SEQ ID n°2.

**12.** Utilisation selon la revendication 11, dans laquelle le polynucléotide comprend un polynucléotide ayant au moins 95% d'identité par rapport au polynucléotide de SEQ ID n°1.

**13.** Utilisation selon les revendications 11 ou 12, dans laquelle le polynucléotide a la séquence polynucléotidique de SEQ ID n°1.